# EUROPEAN PATENT APPLICATION

(11) **EP 1 736 151 A2**
(43) Date of publication of application: **27.12.2006**
(21) Application number: 06016101.5
(22) Date of filing: 06.02.2002
(51) Int. Cl.: A61K 31/18, A61K 31/235, A61K 31/404, A61K 31/4045, A61K 31/435, A61K 31/454, A61K 31/4545, A61K 31/495, A61K 31/506, A61K 31/517, A61K 31/519, A61P 13/02

(54) **Pharmaceutical composition for the therapy of lower urinary tract symptoms**

(30) Priority: 07.02.2001 JP 2001030303
(62) Divisional of application: 02711333.1
(71) Applicant: Astellas Pharma Inc., Tokyo 103-8411 (JP)
(72) Inventor: Shimoyama, Mitsuru, 2-chome, Chuo-ku Tokyo 103-8411 (JP); Watanabe, Takeshi, 2-chome, Chuo-ku Tokyo 103-8411 (JP); Furudate, Naomichi, 2-chome, Chuo-ku Tokyo 103-8411 (JP)
(74) Representative: HOFFMANN EITLE

(57) **Abstract**

A pharmaceutical composition for the therapy of lower urinary tract symptoms where said composition contains an α₁ receptor blocker.

## Description

### Technical Field of the Invention

The present invention relates to a pharmaceutical agent and, more particularly, it relates to a therapeutic agent for lower urinary tract symptoms.

### Background Art

Bladder and urethra which are called lower urinary tracts participate in an urinary function and the function is controlled by three kinds of nerves which are sympathetic nerve, parasympathetic nerve and somatic nerve (pudendal nerve) *(*Rinsho to Kenkyu, 71(5):1180, 1994).

There are various causative diseases for the urinary disturbance and they have been roughly classified into (1) organic obstruction of urethra and (2) abnormality of urination-controlling nerve *(*Brain Nursing, 15, No.1, pages 94-93, 1999).

Examples of (1) organic obstruction of urethra are benign prostatic hyperplasia, urethral stricture, urethral calculus and tumor, etc. With regard to the organic disturbance, the obstruction can be removed by a urologically surgical operation but, because of the risk by the operation and of sequela after the operation, a pharmacotherapy is preferred.

Urinary disfunction associated with benign prostatic hyperplasia is a disease which occurs only in males and the disturbance is caused by both urethral stricture (mechanical obstruction) due to an oppression of enlarged prostate gland and overconstriction (functional obstruction) of prostatic smooth muscle accompanied by an increase in α₁ receptor in enlarged prostate gland (Rinsho Kagaku, 33(12):1542, 1997). With regard to therapeutic agents therefor, preparations of plant and animal extracts and antiandrogens were used firstly. However, preparations of plant and animal extracts have low effectiveness and, moreover, they have no scientific grounds while antiandrogens have a slow onset of effect and a low efficacy and, moreover, they have a disadvantage of causing a side effect of sexual dysfunction such as impotence. Later, α -adrenaline receptor blockers such as tamsulosin which lower intraurethral pressure by blocking the increased α₁ receptor in the prostatic gland were developed and have been commonly used as the drugs having high effectiveness and high safety.

Urinary disturbance due to (2) abnormality of urination-controlling nerve is a urinary disturbance occurring both in males and females caused by disorder of sympathetic nerve which controls the operation of urethra and by that of parasympathetic nerve which controls the operation of bladder, and is generally called neurogenic bladder. Main diseases which result in the neurogenic bladder are encephalopathy such as cerebrovascular accident, Parkinsonism and brain tumor; myelopathy such as myelic injury, spina bifida, ossification of the posterior longitudinal ligament, HAM and tethered cord syndrome; peripheral nerve disturbance such as diabetes mellitus, operation in pelvic cavity and stricture of lumber vertebral canal; and others such as multiple sclerosis and spinocerebellar degeneracy *(*Hyojun Hinyokikigaku [= Textbook of Urology], fifth edition, published in 1998).

With regard to therapeutic agents therefor, there have been attempts for the use of cholinolytics with an object of relaxation of bladder, cholinergics with an object of potentiating the constriction of bladder, and α receptor blockers with an object of lowering the intraurethral pressure. The present applicant has already confirmed that tamsulosin or a salt thereof is clinically effectively for the therapy of neurogenic bladder (PCT/JP99/03343).

On the other hand, in recent years, urinary disturbance which does not correspond to any of apparent organic disturbance and neurological abnormality in lower urinary tracts has been called (3) lower urinary tract symptoms and is being established as a new and third classification for urinary disturbance in addition to (1) organic obstruction of urethra and (2) abnormality of urination-controlling nerve. The causative diseases therefor will be dysuria, urinary neck sclerosis, urinary neck obstruction, urethral syndrome, detrusor-sphincter incoordination, unstable bladder, chronic prostatitis, chronic cystitis, prostatic cancer, Hinman syndrome, Fowler syndrome, psychogenic urinary disturbance, drug-induced urinary disturbance, urinary disturbance due to aging, etc. and urinary disturbance of females is also included therein. However, mechanism of the diseases has not been fully clarified yet and no therapeutic method has been established yet.

At present, there is no pharmaceutical agent in Japan, Europe and America which has been clinically established as a therapeutic agent for lower urinary tract (which is the urinary disturbances as the said third group) having an efficacy. There has been a brisk demand for development of therapeutic agent for the lower urinary tract symptoms.

### Disclosure of the Invention

Under such circumstances, the present inventor has found that α₁ receptor blockers are effective for the therapy of lower urinary tract symptoms which are the urinary disturbances of the third group.

Thus, the present invention relates to a pharmaceutical composition for the therapy of lower urinary tract symptoms where said composition contains an α₁ receptor blocker. The present invention further relates to the use of an α₁ receptor blocker for the manufacture of a therapeutic agent for lower urinary tract symptoms. The present invention furthermore relates to a method for the therapy of lower urinary tract symptoms where said method includes administration of α₁ receptor blocker to a patient.

α₁ receptor blockers have been known to have an α₁ receptor blocking action for the areas of urethra and prostatic gland and has been commonly used as a pharmaceutical agent which lowers the pressure of prostatic gland part of the intraurethral pressure curve and improves the urinary disturbance accompanied by prostatic hypertrophy.

However, there has been no report confirming the efficacy to lower urinary tract symptoms having different onset mechanism. Now the present inventor has clinically confirmed for the first time that α₁ receptor blockers are effective for the therapy of lower urinary tract symptoms.

The present invention will be illustrated in detail as hereunder.

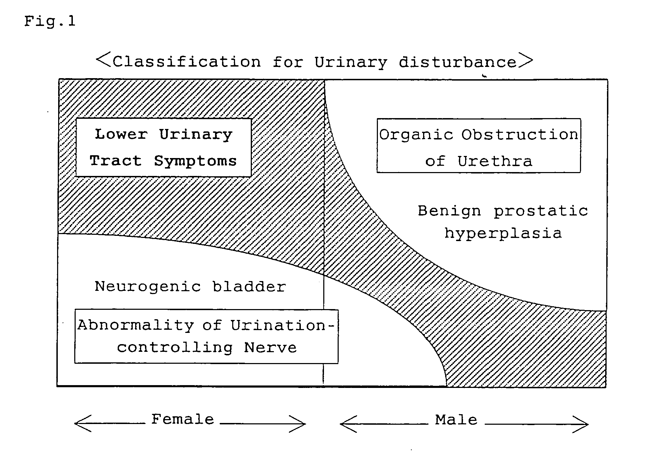

In the present invention, the term "lower urinary tract symptoms" stands for a concept which is a symptom of urinary disturbance due to a functional obstruction of lower urinary tract of both males and females and does not include that which is due to disturbance of nerve controlling the lower urinary tract and that which is due to an organic disturbance of the lower urinary tract. Fig. 1 shows the positioning of the lower urinary tract symptoms in urinary disturbance in terms of classification to clearly show the concept of the said disease. As shown on oblique lines parts, the lower urinary tract symptoms as the object of present invention does not include the diseases for which efficacy of α₁ receptor blockers are reported, such as the urinary disturbance accompanied by prostatic hypertrophy, the urinary disturbance accompanied by neurogenic bladder, etc.

A therapeutic agent for lower urinary tract symptoms is a pharmaceutical agent which treats the lower urinary tract symptoms and/or a pharmaceutical agent which improves the symptom of the lower urinary tract symptoms. A pharmaceutical composition for the therapy of lower urinary tract symptoms contains an effective ingredient which treats the lower urinary tract symptoms and/or improves the symptom of the lower urinary tract symptoms and pharmaceutically acceptable carriers thereof.

In the present invention, α₁ receptor blockers include prazosin, terazosin, bunazosin, urapidil, Moxisylyte, doxazosin, metazosin, indoramin, naftopidil, alfuzosin, fiduxosin, upidosin, KMD-3213(Chemical
name:(-)-1-(3-Hydroxypropyl)-5-[2(R)-[2-[2-(2,2,2-trifluor oethoxy)phenoxy]ethylamino]propyl]indoline-7-carboxamide), SNAP-5089(Chemical
name:5-[N-[3-(4,4-Diphenylpiperidin-1-yl)propyl]carbamoyl] -2,6-dimethyl-4(R)-(4-nitrophenyl)-1,4-dihydropyridine-3-c arboxylic acid methyl ester), AIO-8507L, SL-890591(Chemical name:(2-[3-[4-(5-Chloro-2-methoxyphenyl)piperazin-1-yl]pro pylamino]pyrimidine-4-carboxamide fumarate),
RS-100329(Chemical
Name:5-Methyl-3-[3-[4-[2-(2,2,2-trifluoroethoxy)phenyl]pip erazin-1-yl]propyl] pyrimidine-2,4(1H,3H)-dione hydrochloride), analogue and salt thereof.

The preferable drug in the present invention is the drug which acts selectively on lower urinary tract and has less effect to cardiovascular, that is the drug which has higher affinity for α_{1A} receptor and/or α_{1D} receptor and lowers affinity for α_{1B} receptor. Naftopidil, alfuzosin, fiduxosin, upidosin, KMD-3213, SNAP-5089, SL-890591, RS-100329 or salts thereof are particularly preferred.

The example of salts are salts with inorganic acids such as hydrochloric acid, sulfuric acid, phosphoric acid; salts with organic acids such as fumaric acid, malic acid, citric acid, succinic acid; salts with alkaline metals such as sodium, potassium; and salts with alkaline earth metals such as calcium, magnesium, etc.

The pharmaceutical agent of the present invention can be prepared as oral solid preparations, oral liquid preparations or injection preparations by a conventional method using organic or inorganic carrier, filler and other additives suitable for oral or parenteral administration. Preferred ones are oral solid preparations which can be easily administered by a patient himself/herself and are convenient for preservation and carrying and, to be more specific, they are tablets, diluted powder, granules, fine granules, capsules, pills, etc.

In such solid preparations, the active substance is mixed with at least one inert diluent such as lactose, mannitol, glucose, microcrystalline cellulose, starch, polyvinylpyrrolidone or magnesium metasilicate aluminate. The composition may contain additives other than the inert diluent according to a conventional method and their examples may be binders such as hydroxypropyl cellulose and hydroxypropylmethyl cellulose; lubricants such as magnesium stearate, calcium stearate, polyethylene glycol, starch and talc; disintegrants such as calcium cellulose glycolate; stabilizers such as lactose; solubilizing aids such as glutamic acid and aspartic acid; plasticizers such as Tween 80 and triacetin; and coloring agents such as titanium oxide and iron sesquioxide. If necessary, tablets or pills may be coated with a sugar coat or with a film of a gastric or an enteric substance such as sucrose, gelatin, agar, pectin, hydroxypropyl cellulose and hydroxypropylmethyl cellulose.

The most preferred preparation in the present invention is a sustained-release preparation of a sustained releasing type. The sustained-release preparation may be made into tablets, granules, fine granules or capsules by a known method. The sustained-release preparation is prepared by coating tablets, fine granules, fine granules or capsules with, for example, fat/oil, fatty acid ester of polyglycerol, hydroxypropyl cellulose, etc. by a known method.

The sustained-release preparation disclosed in the Japanese Patent Laid-Open No. Sho-62/9 is particularly preferred. Thus, in each unit preparation, particles which are prepared by granulation after adding an elution suppressor to a mixture of an active compound and not less than 50% by weight of a unit-forming substance in a unit are filled in a capsule to prepare a capsule preparation or to prepare a tablet by a conventional method. With regard to the elution suppressor, a water-insoluble high-molecular substance such as an acrylic polymer, copolymer or cellulose derivative is used and it is appropriate that the elution suppressor is used, for example, in a form of an aqueous suspension, an aqueous emulsion or a solution in a water-containing organic solvent. Examples of the commercially available one are Eudragit L 30D55 (methacrylic acid copolymer LD), Eudragit E 30D (emulsion of copolymer of ethyl acrylate with methyl methacrylate) and Aquacoat ECD-30 (aqueous suspension of ethyl cellulose) and they may be used as elution suppressors either as they are or after diluting with water if necessary.

Dose of α₁ receptor blocker is appropriately decided for each drug and each case taking administering route, symptom of the disease, age and sex of the object to be treated, etc. into consideration. For example, usual dose for an adult by oral administration is about 25 to 150mg/day of naftopidil, about 1 to 12 mg/day of prazosin hydrochloride, about 0.5 to 4 mg/day of terazosin hydrochloride, about 30 to 180 mg/day of urapidil, etc. and that is administered *per os* after meals once daily.

Incidentally, the pharmaceutical agent of the present invention is well effective by its sole administration but it is also possible that a cholinergic agent, a cholinolytic agent or other central nerve drug is used together either simultaneously or with a time interval.

### Brief Explanation of the Drawings

Fig. 1 shows the positioning of the lower urinary tract symptoms in urinary disturbance in terms of classification.

### Best Mode for Carrying Out the Invention

The present invention will now be illustrated in more detail by way of Examples and Test Examples as hereunder although the present invention is not limited to those Examples, etc.

### Example 1.

5 g of tamsulosin hydrochloride and 470 g of crystalline cellulose were well mixed, 500 g of a mixture of 83.3 g of Eudragit L30D-55 (25 g as a solid) with water were added thereto and the mixture was granulated using a high-speed stirring granulator. The resulting particles were in a spherical shape having a particle size of 0.1-1.5 mm where most of them were 0.2-1.0 mm.

The resulting particles were mixed with talc and magnesium stearate and filled in capsules to prepare capsule preparations (containing 0.2 mg of tamsulosin hydrochloride in a capsule).

### Examples 2-6.

The same process as in Example 1 was conducted whereby the particles manufactured according to the formulations of Table 1 were made into capsule preparations.

**Table 1 (unit: grams)**

| Example Number | Tamsulosin Hydrochloride (g) | Crystalline Cellulose (g) | Eudragit L30D-55 (Solid Content) (g) |
|---|---|---|---|
| 2 | 5 | 445 | 166.6 (50) |
| 3 | 5 | 395 | 333.3 (100) |
| 4 | 5 | 482.5 | 41.7(12.5) |
| 5 | 2.5 | 472.5 | 83.3 (25) |
| 6 | 1.25 | 473.75 | 83.3 (25) |

### Example 7.

5 g of tamsulosin hydrochloride, 420 g of crystalline cellulose and 50 g of magnesium stearate were well mixed, a mixture of 83.3 g of Eudragit L30D-55 (25 g as a solid) with water were added thereto and the mixture was kneaded followed by granulating by a centrifugal fluid granulator. The resulting particles were in a spherical shape having a particle size of 0.1-1.5 mm where most of them were 0.2-1.0 mm.

The resulting particles were mixed with talc and magnesium stearate and filled in capsules to prepare capsule preparations (containing 0.2 mg of tamsulosin hydrochloride in a capsule).

### Examples 8-10.

The same process as in Example 7 was conducted whereby the particles manufactured according to the formulations of Table 2 were made into capsule preparations.

**Table 2 (unit: grams)**

| Example Number | Tamsulosin Hydrochloride | Crystalline Cellulose | Magnesium Stearate | Eudragit L30D-55 (Solid Content) |
|---|---|---|---|---|
| 8 | 5 | 460 | 10 | 83.3 (25) |
| 9 | 5 | 445 | 25 | 83.3 (25) |
| 10 | 2.5 | 462.5 | 10 | 83.3 (25) |

### Example 11.

80 g of hydrogenated castor oil was melted, 10 g of tamsulosin hydrochloride and 30 g of hydroxypropyl cellulose having a low degree of substitution were dispersed therein and the dispersion was made into fine particles by means of a spray condyling. The resulting fine particles (60 g) were well mixed with 440 g of crystalline cellulose, 500 g of water were added thereto and the mixture was granulated using a centrifugal fluid granulator.

The resulting particles were mixed with talc and magnesium stearate and filled in capsules to prepare capsule preparations.

### Example 12.

The tablet prepared according to the formulations of Table 3.

**Table 3 (unit: grams)**

| | |
|---|---|
| Naftopidil | 25.0 |
| Lactose monohydrate | 110.0 |
| Porvinylpyrrolidone | 3.0 |
| Microcrystalline Cellulose | 15.0 |
| Highly dispersed silicon dioxide | 1.5 |
| Poly-(O-carboxymethyl)-starch Sodium Salt | 4.0 |
| Magnesium stearate | 1.5 |
| Total | 160.0 |

### Example 13.

The tablet in a hard gelatin capsule for a once-daily oral administration prepared according to the formulations of Table 4.

**Table 4 (unit: grams)**

| Tablet No.1 | |
|---|---|
| Alfuzosin hydrochloride | 3.3 |
| Microcrystalline cellulose | 30.0 |
| Dicalcium phosphate dihydrate | 42.7 |
| Hydrogenated caster oil | 18.0 |
| Polyvinylpyrrolidone | 5.0 |
| Magnesium stearate | 1.0 |
| Tablet No.2 | |
| Alfuzosin hydrochloride | 3.3 |
| Lactose | 69.4 |
| Microcrystalline cellulose | 17.8 |
| Polyvinylpyrrolidone | 5.0 |
| Sodium carboxymethylstarch | 4.0 |
| Magnesium stearate | 0.5 |

| Coating of the tablet No.2 | |
|---|---|
| Methacrylic acid copolymer | 75.7 |
| Diacetylated monoglycerides | 7.5 |
| Talc | 16.8 |

Test Example 1 . Clinical Test to Patients Suffering from Lower Urinary Tract Symptoms (a four-week test)

A clinical test was carried out under the following conditions using the patients suffering from lower urinary tract symptoms. (cf. Chapter 1: Indexes for Evaluation of Prostatic Hypertrophy in "Guideline for Clinical Test for Urinary Disturbance" published by Iyaku Tosho Shuppan)

Subjects: four patients diagnosed as lower urinary tract symptoms, i.e. urinary disturbance without clear organic or neurological abnormality in lower urinary tract (3 males and 1 female).

Test drug and administering method: One capsule containing 0.2 mg of tamsulosin hydrochloride was orally administered once daily after breakfast.

### Test period: four weeks (28 days)

Observed items: Evaluations and measurements were made for the following items before and after the administration.

### (1) Total score for subjective symptoms

Questioning was done to the patients for the following items and the total score was obtained.

### <1> Feel of residual urine

"How often do you have a sensation of not emptying your bladder completely after you finished urinating?"
0: not at all
1: not so often
2: sometimes
3: about half the time
4: often
5: almost always

### <2> Urination within two hours

"How often do you have to urinate again less than two hours after you finished urinating?"
0: not at all
1: not so often
2: sometimes
3: about half the time
4: often
5: almost always

### <3> Break of urinary stream

"How often do you find you stopped and started again several times when you urinated?"
0: not at all
1: not so often
2: sometimes
3: about half the time
4: often
5: almost always

### <4> Urgency

"How often do you find it difficult to postpone urination?"
0: not at all
1: not so often
2: sometimes
3: about half the time
4: often
5: almost always

### <5> Force of urinary stream

"How often do you have a weak urinary stream?"
0: not at all
1: not so often
2: sometimes
3: about half the time
4: often
5: almost always

### <6> Straining upon urination

"How often do you have to push or strain to begin urination?"
0: not at all
1: not so often
2: sometimes
3: about half the time
4: often
5: almost always

### <7> Frequency of urination at night

"How many times do you most typically get up to urinate from the time you go to bed at night until the time you get up in the morning?"
0: none
1: one times
2: two times
3: three times
4: four times
5: five or more times

The results of the observed items before and after the test are shown in Table 3.

**Table 3**

| sex | age | Total score for subjective symptoms | | |
|---|---|---|---|---|
| | | before the test | after the test | Changing Rate (%) |
| female | 54 | 15 | 11 | -26.7 |
| male | 69 | 26 | 9 | -65.4 |
| male | 62 | 25 | 13 | -48.0 |
| male | 47 | 26 | 20 | -23.1 |
| avg. | 58.0 | 23.0 | 13.3 | -40.8 |

| | | | | |
|---|---|---|---|---|
| *) Changing Rate (%) = [(Total score for subjective symptoms after the test) ― (Total score for subjective symptoms before the test)] ÷ (Total score for subjective symptoms before the test) × 100 | | | | |

### (2) QOL Index

With regard to an index for quality of life (QOL), a questioning that "How do you think if the present urinary condition continues in future as well?" was done as a total diagnosis concerning the urinary disturbance and any of the following evaluations was collected.
0: quite satisfactory
1: satisfactory
2: generally satisfactory
3: neither satisfactory nor unsatisfactory
4: a bit unsatisfactory
5: unsatisfactory
6: quite unsatisfactory

The result was that the mean value for the patients before the administration was 4.8 while that after the administration was 3. 8 whereby there was an improvement of one point in average in terms of the QOL index.

From the above result, tamsulosin hydrochloride showed improvements in (1) the total score for subjective symptom and (2) the QOL index for the patients suffering from lower urinary tract symptoms whereby tamsulosin hydrochloride was confirmed to be effective as a therapeutic agent for lower urinary tract symptoms.

Test Example 2. Clinical Test to Patients Suffering from Lower Urinary Tract Symptoms (a twelve-week test)

A clinical test was carried out under the following conditions using the patients suffering from lower urinary tract symptoms.

Subjects: eighteen patients diagnosed as lower urinary tract symptoms, i. e. urinary disturbance without clear organic or neurological abnormality in lower urinary tract (15 males and 3 female, age:57.2±14.2).

Test drug and administering method: One capsule containing 0.2 mg of tamsulosin hydrochloride was orally administered once daily after breakfast for four weeks and, after that, the dose after 4 weeks was controlled in consideration of the following criterion, and the dose was orally administered once daily after breakfast.

### <Criterion of Dose after 4 weeks>

| side effects | Changing Rate of Total score for subjective symptoms in 4 weeks | dose of after 4 weeks |
|---|---|---|
| yes | | 0.2mg/day |
| no | Changing Rate of Total score for subjective symptoms ≦ -25.0% | |
| | Changing Rate of Total score for subjective symptoms ≧ -24.9% | 0.4mg/day |

### Test period: twelve weeks (84 days)(4 weeks(28 days) for 8 males)

Observed items: Evaluations and measurements were made for the following items before and after the administration.

### (1) Total score for subjective symptoms

Total scores for the following <1>-<7> before and after the administration were obtained in the same way as in Test Example 1.

### <1> Feel of residual urine, <2> Urination within two hours, <3> Break of urinary stream, <4> Urgency, <5> Force of urinary stream, <6> Straining upon urination and <7> Frequency of urination at night.

### (2) QOL Index

"How do you think if the present urinating state continues in future as well?"
0: quite satisfactory
1: satisfactory
2: generally satisfactory
3: neither satisfactory nor unsatisfactory
4: a bit unsatisfactory
5: unsatisfactory
6: quite unsatisfactory

### (3) Test on Functions

<1> maximum urinary stream rate
<2> average urinary stream rate

The results of the observed items after the test are shown in Table 4.

**Table 4**

| the observed items | | after 4 week | after 12 week |
|---|---|---|---|
| Changing Rate of Total score for subjective symptoms | male | -43.0±26.5% | -47.0±35.6% |
| | | (n=15) | (n=7) |
| | Female | -30.5±16.9% | -37.4±26.8% |
| | | (n=3) | (n=3) |
| | Total | -40.9±25.2% | -44.6±32.1% |
| | | (n=18) | (n=10) |
| Changing Amt of QOL Index | male | -1.6±1.8 | -2.9±1.9 |
| | | (n=15) | (n=7) |
| | Female | -1.3±1.2 | -3.0±1.7 |
| | | (n=3) | (n=3) |
| | Total | -1.6±1.7 | -2.9±1.7 |
| | | (n=18) | (n=10) |
| Changing Amt of Maximum urinary stream rate | male | +2.9±7.4ml/s | +7.8±4.3ml/s |
| | | (n=15) | (n=7) |
| | Female | +3.8±1.9ml/s | +6.2±6.0ml/s |
| | | (n=3) | (n=3) |
| | Total | +3.1±6.7ml/s | +7.3±4.6ml/s |
| | | (n=18) | (n=10) |
| Changing Amt of Average urinary stream rate | male | +1.9±3.6ml/s | +4.6±2.2ml/s |
| | | (n=15) | (n=7) |
| | Female | +2.2±1.8ml/s | +2.1±2.8ml/s |
| | | (n=3) | (n=3) |
| | Total | +2.0±3.3ml/s | +3.8±2.5ml/s |
| | | (n=18) | (n=10) |

The results after 12 weeks in this test are decided by "Guideline for Clinical Test for Urinary Disturbance" that the effective rate(Degree of Improvement as a whole is more than "Useful") is 70.0%.

From the above result, tamsulosin hydrochloride showed improvements in (1) the total score for subjective symptom, (2) the QOL index and (3) test on functions for the patients suffering from lower urinary tract symptoms whereby tamsulosin hydrochloride was confirmed to be effective as a therapeutic agent for lower urinary tract symptoms.

### Industrial Applicability

In accordance with the present invention, there is provided a clinically effective and good therapeutic agent for lower urinary tract symptoms.

## Claims

1. A pharmaceutical composition for the therapy of lower urinary tract symptoms where said composition contains an α₁ receptor blocker.

2. A pharmaceutical composition for the therapy of lower urinary tract symptoms according to claim 1 in which said α₁ receptor blocker is selected from naftopidil, alfuzosin, fiduxosin, upidosin, SNAP-5089, SL-890591, RS-100329 and salts thereof.

3. The use of an α₁ receptor blocker for the manufacture of a therapeutic agent for lower urinary tract symptoms.

4. A method for the therapy of lower urinary tract symptoms where said method includes administration of an α₁ receptor blocker to a patient.
